(19) Europäisches Patentamt / European Patent Office / Office européen des brevets

(11) **EP 3 927 690 B1**

(12) **EUROPEAN PATENT SPECIFICATION**

(45) Date of publication and mention
of the grant of the patent:
**18.09.2024 Bulletin 2024/38**

(21) Application number: **20707012.9**

(22) Date of filing: **17.02.2020**

(51) International Patent Classification (IPC):
*C07D 213/89* *(2006.01)*     *A61K 8/49* *(2006.01)*
*A61P 31/10* *(2006.01)*     *A61Q 5/00* *(2006.01)*
*C07C 215/08* *(2006.01)*

(52) Cooperative Patent Classification (CPC):
**A61P 31/10; A61K 8/4926; A61Q 5/006;**
**C07C 215/08; C07D 213/89**

(86) International application number:
**PCT/EP2020/054131**

(87) International publication number:
**WO 2020/169544 (27.08.2020 Gazette 2020/35)**

(54) **PIROCTONE OLAMINE RECRYSTALLIZATION**

PIROCTON OLAMIN-REKRISTALLISATION

RECRISTALLISATION DE PIROCTONE OLAMINE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **19.02.2019 IN 201911006542**
**20.12.2019 EP 19218774**

(43) Date of publication of application:
**29.12.2021 Bulletin 2021/52**

(73) Proprietor: **Clariant International Ltd**
**4132 Muttenz (CH)**

(72) Inventors:
• **ENDRES, Andreas**
**89264 Weißenhorn (DE)**

• **METHA, Hiten**
**Ahmedabad 380015 Gujarat (IN)**
• **MULLER, Thierry**
**9026 Ettelbruck (LU)**

(74) Representative: **Clariant Produkte (Deutschland)**
**GmbH**
**Patent Management**
**Industriepark Höchst, G 860**
**65926 Frankfurt am Main (DE)**

(56) References cited:
**EP-A2- 0 710 650     DE-C1- 3 626 210**

EP 3 927 690 B1

**Description**

The present invention relates to a process for recrystallizing piroctone olamine and to piroctone olamine crystals which may be obtained thereby. The present invention further relates to a process for preparing piroctone olamine crystals and to piroctone olamine crystals which may be obtained thereby.

With reference to DE 2 234 009 A1 and DE 1 795 270 A1, 1-Hydroxy-4-methyl-6-(2,4,4-trimethyl)-pentyl-2(1H)-pyridone, 2-aminoethanol salt, also known as piroctone ethanolamine or piroctone olamine, is an anti-fungal active agent which is effective against the causes of dandruff. It is known to include piroctone olamine in personal care products, such as shampoos. DE 1 795 270 A1 also describes a method of making piroctone olamine.

Piroctone olamine exists in the form of crystals which may be added to personal care products. Commercially available piroctone olamine made by processes such as those referred to above, typically have primary crystals with a width/length ratio of about 1:7 and a median diameter ($d_{50}$) of about 100 micrometers. The primary crystals are those formed during the crystallization process, but prior to further processing and bulk handling steps. After such further processing and handling in bulk quantities, the crystals may change dimensions. Such bulk quantities of piroctone olamine crystals may gather together to form clumps. Clumping phenomena may give rise to difficulties when handling and processing the bulk crystals.

EP 0 710 650 A2 discloses a synthesis of piroctone olamine where piroctone olamine is isolated from ethyl acetate.

DE 36 26 210 C1 discloses a synthesis of piroctone olamine where piroctone olamine is isolated from ethyl acetate.

CN 1907971 A relates to a process for manufacturing the anti-fungal material, ciclopirox olamine. This document discusses recrystallization, but it does not touch upon the problem of clumping.

It is with this background that the present invention has been devised.

In this document, including in all embodiments of all aspects of the present invention, the following definitions apply unless specifically stated otherwise.

In relation to the particle size distribution measures used herein, $d_{50}$, d(50) or D50, the median, is defined as the diameter for which where half of the population lies below this value. Similarly, 10 percent of the population lies below the $d_{10}$, d(10) or D10 diameter.

All percentages are by weight (w/w) of the total composition. All ratios are weight ratios. "wt.%" means percentage by weight. References to 'parts' e.g. a mixture of 1 part X and 3 parts Y, is a ratio by weight. "QS" or "QSP" means sufficient quantity for 100% or for 1 00g. +/- indicates the standard deviation. All ranges are inclusive and combinable. The number of significant digits conveys neither a limitation on the indicated amounts nor on the accuracy of the measurements. All numerical amounts are understood to be modified by the word "about". All measurements are understood to be made at 23°C and at ambient conditions, where "ambient conditions" means at 1 atmosphere (atm) of pressure and at 50% relative humidity. "Relative humidity" refers to the ratio (stated as a percent) of the moisture content of air compared to the saturated moisture level at the same temperature and pressure. Relative humidity can be measured with a hygrometer, in particular with a probe hygrometer from VWR® International. Herein "min" means "minute" or "minutes". Herein "mol" means mole. Herein "g" following a number means "gram" or "grams" and "kg" means "kilogram" or "kilograms". Herein, "comprising" means that other steps and other ingredients can be in addition. Embodiments and aspects described herein may comprise or be combinable with elements, features or components of other embodiments and/or aspects despite not being expressly exemplified in combination, unless an incompatibility is stated. "In at least one embodiment" means that one or more embodiments, optionally all embodiments or a large subset of embodiments, of the present invention has/have the subsequently described feature. "Molecular weight" or "M.Wt." or "MW" and grammatical equivalents mean the number average molecular weight.

According to a first aspect of the invention, a process for the recrystallisation of piroctone olamine is provided, comprising:

a) Dissolving piroctone olamine in a solvent comprising alcohol, wherein dissolving takes place at a dissolution temperature, which is a temperature above 50 degrees Celsius and below the boiling point of the alcohol, wherein the solvent comprises 90%wt or more of alcohol, and wherein the alcohol comprised in the solvent is selected from the group consisting of ethanol, isopropanol and mixtures thereof;
b) Cooling the solution from the dissolution temperature to a temperature between 1 and 15 degrees Celsius for a period up to 10 hours, preferably from 10 minutes to 10 hours, more preferably from 1 to 7 hours, even more preferably from 1 to 5 hours;
c) Recovering crystals of piroctone olamine.

According to a second aspect of the invention, a process for the preparation of piroctone olamine crystals is provided, comprising:

a) Dispersing piroctone olamine in a solvent comprising alcohol, wherein dispersing takes place at a dispersion temperature, which is a temperature above 50 degrees Celsius and below the boiling point of the alcohol, wherein the solvent comprises 90%wt or more of alcohol, and wherein the alcohol comprised in the solvent is selected from the group consisting of ethanol, isopropanol and mixtures thereof;

b) Cooling the dispersion from the dispersion temperature to a temperature between 1 and 15 degrees Celsius for a period up to 15 hours, preferably from 10 minutes to 15 hours, more preferably from 1 to 12 hours;

c) Recovering crystals of piroctone olamine.

The recrystallization process of the first aspect of the invention and the preparation process of the second aspect of the invention give rise to crystals which have different dimensions from the crystals of the starting product. Bulk quantities of these differently dimensioned crystals are surprisingly observed to be less liable to clumping. These crystals are subject of the third aspect of the invention.

According to the first aspect of the invention 1.5 to 4kg, preferably 2kg, of solvent are used per kilogram of piroctone olamine.

In a particular embodiment, crystals of piroctone olamine are added for seeding. A person skilled in the art is aware of the concept of seeding / using seed crystals. Preferably, crystals of piroctone olamine for seeding are added after step a). Also preferably, crystals of piroctone olamine for seeding are added before or during step b). Preferably, crystals of piroctone olamine for seeding are added in an amount of 0.1 to 3.0 wt.%, more preferably 0.3 to 1.0 wt.%, particularly preferably 0.5 wt.%, based on the amount of piroctone olamine used in step a).

According to the first aspect of the invention cooling may take place in two steps:

i. In a first cooling step, cooling the solution from the dissolution temperature to a temperature between 20 and 40 degrees Celsius for a first cooling period which lasts up to 5 hours;

ii. In a second cooling step, cooling the solution from a temperature between 20 and 40 degrees Celsius to a temperature between 1 and 15 degrees Celsius for a second cooling period which lasts up to 5 hours. Preferably, the second cooling period lasts for 3 hours or less.

The alcohol comprised in the solvent is selected from the group consisting of ethanol, isopropanol and mixtures thereof. Preferably, the alcohol comprises isopropanol.

The solvent may additionally comprise a material selected from ethyl acetate, isopropyl acetate, butyl acetate, dichloromethane, chloroform, n-hexane, benzene, toluene, acetone, butanone, diisopropyl ether, methyl tert-butyl ether, tetrahydrofuran, dioxane and mixtures thereof.

The solvent comprises 90%wt or more of alcohol. More preferably, the solvent consists of alcohol. More preferably still, the solvent consists of isopropanol.

According to the first aspect of the invention, in a) the dissolution temperature is from 60 to 82.5 degrees Celsius, preferably from 70 to 75 degrees Celsius. Furthermore, the mixture of piroctone olamine and solvent is maintained at the dissolution temperature until the piroctone olamine has completely dissolved in the solvent. A clear solution indicates complete dissolution.

According to the second aspect of the invention 1 to 3kg, preferably 1.0 to 1.5kg, more preferably 1.2 to 1.4kg, particularly preferably 1.33kg, of solvent are used per kilogram of piroctone olamine.

In a particular embodiment, crystals of piroctone olamine are added for seeding. Preferably, crystals of piroctone olamine for seeding are added after step a). Also preferably, crystals of piroctone olamine for seeding are added before or during step b). Preferably, crystals of piroctone olamine for seeding are added in an amount of 0.1 to 3.0 wt.%, more preferably 0.3 to 1.0 wt.%, particularly preferably 0.5 wt.%, based on the amount of piroctone olamine used in step a).

According to the second aspect of the invention cooling may take place in two steps:

i. In a first cooling step, cooling the dispersion from the dispersion temperature to a temperature between 20 and 40 degrees Celsius for a first cooling period which lasts up to 10 hours;

ii. In a second cooling step, cooling the dispersion from a temperature between 20 and 40 degrees Celsius to a temperature between 1 and 15 degrees Celsius for a second cooling period which lasts up to 5 hours. Preferably, the second cooling period lasts for 3 hours or less.

The alcohol comprised in the solvent is selected from the group consisting of ethanol, isopropanol and mixtures thereof. Preferably, the alcohol comprises isopropanol.

The solvent may additionally comprise a material selected from ethyl acetate, isopropyl acetate, butyl acetate, dichloromethane, chloroform, n-hexane, benzene, toluene, acetone, butanone, diisopropyl ether, methyl tert-butyl ether, tetrahydrofuran, dioxane and mixtures thereof.

The solvent comprises 90%wt or more of alcohol. More preferably, the solvent consists of alcohol. More preferably still, the solvent consists of isopropanol.

According to the second aspect of the invention, in a) the dispersion temperature is from 60 to 82.5 degrees Celsius, preferably from 70 to 75 degrees Celsius.

According to the third aspect of the invention, piroctone olamine crystals are provided, having an average width to length ratio of greater than 1:4, obtainable by a process for the recrystallisation of piroctone olamine as defined herein or by a process for the preparation of piroctone olamine crystals as defined herein.

For completeness, since a ratio is being referred to, by saying that the ratio is "greater than" a certain value means that the second number (after the colon) is less than the given number, that is less than "4" in the present case.

As discussed above, commercially available piroctone olamine made by processes such as those referred to above, typically have primary crystals with a width/length ratio of about 1:7. The primary crystals are those formed during the crystallization process, but prior to further processing and bulk handling steps. During such processing and bulk handling steps, the primary crystals, which are brittle, may change shape, but are found to have an average width/length of significantly less than 1:4. As explained, crystals having such dimensions may suffer, in bulk quantities, to clumping phenomena.

Preferably, the piroctone olamine crystals have an average width to length ratio of greater than 1:4 and smaller than 1:2.

In a preferred embodiment of the third aspect of the invention, the piroctone olamine crystals have $d_{50}$ of greater than 110 micrometres and preferably greater than 140 micrometres. In a more preferred embodiment, the piroctone olamine crystals have $d_{10}$ greater than 30 micrometers and preferably greater than 50 micrometres.

Preferably, the piroctone olamine crystals have $d_{50}$ of greater than 110 micrometres and less than 400 micrometres. More preferably, the piroctone olamine crystals have $d_{50}$ of greater than 140 micrometres and smaller than 350 micrometres. Even more preferably, the piroctone olamine crystals have $d_{50}$ of greater than 140 micrometres and smaller than 300 micrometres.

In a preferred embodiment, the piroctone olamine crystals have $d_{50}$ of greater than 170 micrometres and smaller than 350 micrometres. In a particularly preferred embodiment, the piroctone olamine crystals have $d_{50}$ of greater than 250 micrometres and smaller than 300 micrometres.

Preferably, the piroctone olamine crystals have $d_{10}$ greater than 30 micrometers and smaller than 120 micrometres. More preferably, the piroctone olamine crystals have $d_{10}$ greater than 50 micrometers and smaller than 100 micrometres. In a particularly preferred embodiment, the piroctone olamine crystals have $d_{10}$ greater than 70 micrometers and smaller than 100 micrometres.

The piroctone olamine crystals are obtainable by a process for the recrystallisation of piroctone olamine as defined herein.

The piroctone olamine crystals are obtainable by a process for the preparation of piroctone olamine crystals as defined herein.

The invention relates to piroctone olamine crystals, obtainable by a process for the recrystallisation of piroctone olamine as defined herein.

The invention relates to piroctone olamine crystals, obtainable by a process for the preparation of piroctone olamine crystals as defined herein.

The invention will now be further described with reference to the accompanying drawings, in which:

Figure 1 illustrates a microscope image of some recrystallized piroctone olamine crystals. The image is divided into 4 parts using a reticle scale in preparation for making width and length measurements in the fashion described below.

Figure 2 illustrates the volumetric distribution density of particles, q (left hand y-axis) and the volumetric cumulative distribution Q(r) (right hand y-axis) versus diameter (x-axis) of particles of piroctone olamine which have not been recrystallized according to the invention.

Figure 3 illustrates the volumetric distribution density of particles, q (left hand y-axis) and the volumetric cumulative distribution Q(r) (right hand y-axis) versus diameter (x-axis) of particles of piroctone olamine according to Example 3, which have been subject to a recrystallization according to the invention.

Figure 4 illustrates a comparison of time consolidations of piroctone olamine, which has been recrystallized using the method of the invention (left hand graph) and non-recrystallized piroctone olamine (right hand graph).

Particle Size Distribution Measurement Method

A Horiba LA-950 particle size analyzer was used for measuring the diamter, the volumetric distribution density and the volumetric cumulative distribuition of the product. The analyzer uses a laser diffraction method (ISO 13320:2009, Fraunhofer Diffraction Method) to measure the distribution and is based on the direct proportionality of the intensity of light scattered by a particle, to the diameter. Furthermore the scattering angle is inversely proportional to the diameter and vice versa.

In preparation for the analysis, the required amount of product is placed on a sieve with a mesh size of 1 mm. The product is sieved with an amplitude of 1.5 mm for 3 minutes.

The required amount of sieved product is added to the gutter of the dry dispersion unit.

Three measurements were made in the HORIBA LA-950 particle size analyzer with the following parameters:

- Gutter starting value 85 -110 (unit-less), automatic control
- Dispersing pressure 0.3 MPa

The three measurements are combined with the software to form an averaged measurement. For analysis the focus is on $d_{10}$ and $d_{50}$ volume fractions.

Measurement the Width/Length Ratio of the Piroctone Olamine Crystals

A small amount of sieved product (see above) is spread on a Petri dish with a spatula.

Under a microscope, a position is sought in which isolated particles are clearly visible. The microscope used was a Keyence VHX 2000 series digital micoscope with a VH-Z20W zoom lens, using a VHX-S90BE free angle observation system. The microscope does not form part of the invention and a skilled person could select suitable alternative microscopes.

The limits are set for the depth of field and an image in the appropriate magnification with the depth of field function of the microscope made.

Pictures are taken at the following magnifications x50, x100, 150, x200 in order to obtain an overall impression of the bulk crystals.

A position on the Petri dish is needed in which an area of 3x3 images can be made with as many individual particles as possible.

A 3x3 image is taken at a magnification of x200.

The image is divided into 4 parts using a reticle scale. In each quarter 5 representative particles are selected (20 particles in total). For each of these 20 particles, the width (w) and length (L) and the w/L ratio are determined. The average w/L ratio for all the particles is then calculated, which is the sum of the measured w/L divided by the number of particles ($\Sigma$w/L)/20).

Flowability Measurement

Clumping of the crystals may be regarded as a low degree of flowability. In order to obtain an objective measurement of clumping, therefore, the crystals' flowability may be measured. The skilled person would be aware of other ways to characterize clumping.

The flowability of a bulk solid may be characterized by its unconfined yield strength, $\sigma_c$, in dependence on consolidation stress, $\sigma_1$, and storage period, t. Usually the ratio $ff_c$ of consolidation stress, $\sigma_1$, to unconfined yield strength, $\sigma_c$, is used to characterize flowability numerically:

$$ff_c = \sigma_1 / \sigma_c$$

The larger $ff_c$ is, i.e., the smaller the ratio of the unconfined yield strength, $\sigma_c$, to the consolidation stress, $\sigma_1$, the better a bulk solid flows. Flow behavior is defined as follows:

$ff_c$ of less than 1, not flowing
$ff_c$ from 1 to less than 2, very cohesive
$ff_c$ from 2 to less than 4, cohesive
$ff_c$ from 4 to less than 10, easy flowing
$ff_c$ of greater than 10, free flowing

The parameter $ff_c$ may be generated using a ring sheer test in the fashion described by Schulze, D (2009) "Pulver und Schüttgüter", 2nd Edition, Springer, Berlin. This method does not form part of the present invention and is merely referred to as one way to characterize flowability in order to demonstrate the effect of the more flowable nature of the recrystallized crystals according to the invention. The skilled person is aware of other ways to characterize flowability.

LABORATORY SYNTHESIS EXAMPLES

Example 1

Raw materials:

| Raw material | Quantity | Equivalents |
|---|---|---|
| Piroctone Olamine | 100 kg | 1.0 |
| Ethanol | 200 kg Two batches of 37 L | 2.74 |

Procedure:

1. Clean and dry the reactor.
2. Charge ethanol (200 kg) at 25-30 degrees Celsius.
3. Charge piroctone olamine (100 Kg) at 25-30 degrees Celsius.
4. Heat the mass to 70 degrees Celsius.
5. Maintain at 70-75 degrees Celsius for 30 minutes.
6. Check for dissolution (clear solution should be observed).
7. If incomplete dissolution, maintain at 70-75 degrees Celsius for another 30 minutes and check for clarity.
8. Gradually cool the mass to 25-30 degrees Celsius over a period of 3-4 hrs.
9. Cool the mass to 8-10 degrees Celsius over a period of 2 hours.
10. Maintain at 8-10 degrees Celsius for 1 hour.
11. Centrifuge at 8-10 degrees Celsius, wash centrifuge with chilled (at 5-10 degrees Celsius) ethanol (wash twice with 37 L).
12. Spin dry for 30 min.
13. Unload the wet in to poly bags and weigh the material.
14. Dry the material in hot air oven below 45 degrees Celsius for 4 hours.
15. Dry the material at 60 degrees Celsius until 0.3%wt liquid.
16. 79.1kg of wet product / 77.4kg dry product was obtained.

Example 2

Raw materials:

| Raw material | Quantity | Equivalents |
|---|---|---|
| Piroctone Olamine | 100 kg | 1.0 |
| Isopropyl alcohol | 200 kg Two batches of 37 L | 2.74 |

Procedure:

1. Clean and dry the reactor.
2. Charge Isopropyl alcohol (200 kg) at 25-30 degrees Celsius.
3. Charge piroctone olamine (100 Kg) at 25-30 degrees Celsius.
4. Heat the mass to 70 degrees Celsius.

5. Maintain at 70-75 degrees Celsius for 30 minutes.

6. Check for dissolution (clear solution should be observed).

7. If incomplete dissolution, maintain at 70-75 degrees Celsius for another 30 minutes and check for clarity.

8. Gradually cool the mass to 8-10°C over a period of 4-5 hrs.

9. Maintain at 8-10 degrees Celsius for 1 hour.

10. Centrifuge at 8-10 degrees Celsius, wash centrifuge with chilled (at 5-10 degrees Celsius) isopropyl alcohol (wash twice with 37 L).

11. Spin dry for 30 min.

12. Unload the wet in to poly bags and weigh the material.

13. Dry the material in hot air oven below 45 degrees Celsius for 4 hours.

14. Dry the material at 60 degrees Celsius until 0.3%wt liquid.

15. 92.5 kg of wet product / 90.5 kg dry product was obtained.

Example 3

Raw materials:

| Raw material | Quantity | Equivalents |
|---|---|---|
| Piroctone Olamine | 100 kg | 1.0 |
| Isopropyl alcohol | 200 kg Two batches of 37 L | 2.74 |

Procedure:

1. Clean and dry the reactor.

2. Charge Isopropyl alcohol (200 kg) at 25-30 degrees Celsius.

3. Charge piroctone olamine (100 Kg) at 25-30 degrees Celsius.

4. Heat the mass to 70 degrees Celsius.

5. Maintain at 70-75 degrees Celsius for 30 minutes.

6. Check for dissolution (clear solution should be observed).

7. If incomplete dissolution, maintain at 70-75 degrees Celsius for another 30 minutes and check for clarity.

8. At 65-70°C add 500 g of piroctone olamine crystals for seeding.

9. Gradually cool the mass to 25-30 degrees Celsius over a period of 3-4 hrs.

10. Cool the mass to 8-10 degrees Celsius over a period of 2 hours.

11. Maintain at 8-10 degrees Celsius for 1 hour.

12. Centrifuge at 8-10 degrees Celsius, wash centrifuge with chilled (at 5-10 degrees Celsius) isopropyl alcohol (wash twice with 37 L).

13. Spin dry for 30 min.

14. Unload the wet in to poly bags and weigh the material.

15. Dry the material in hot air oven below 45 degrees Celsius for 4 hours.

16. Dry the material at 60 degrees Celsius until 0.3%wt liquid.

17. 93.0kg of wet product / 91.0kg dry product was obtained.

Results

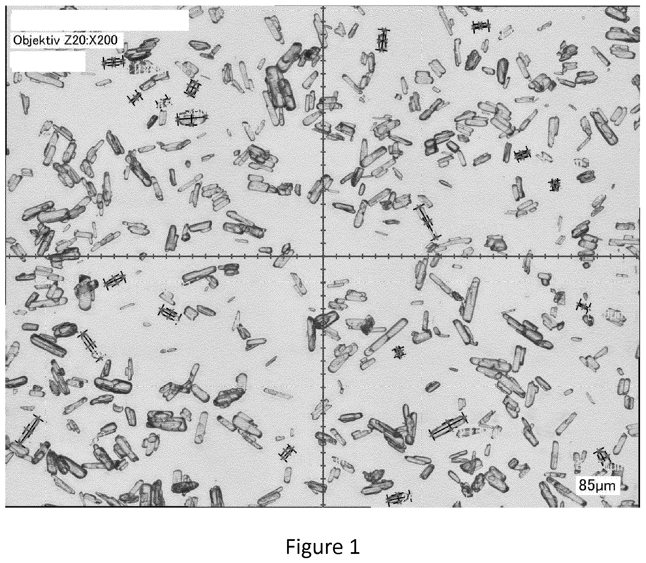

Figure 1 illustrates recrystallized crystals of piroctone olamine made according to Example 3, at a magnification of x200. The image is divided into four for measurement purposes as discussed above and indicates particles whose dimensions have been measured. In total, the w/L of each of 20 particles was measured and the average w/L value was determined. The measurements taken from individual particles are shown. The same procedure had previously been performed on the starting material of piroctone olamine crystals which had not been subject to a recrystallization according to the invention.

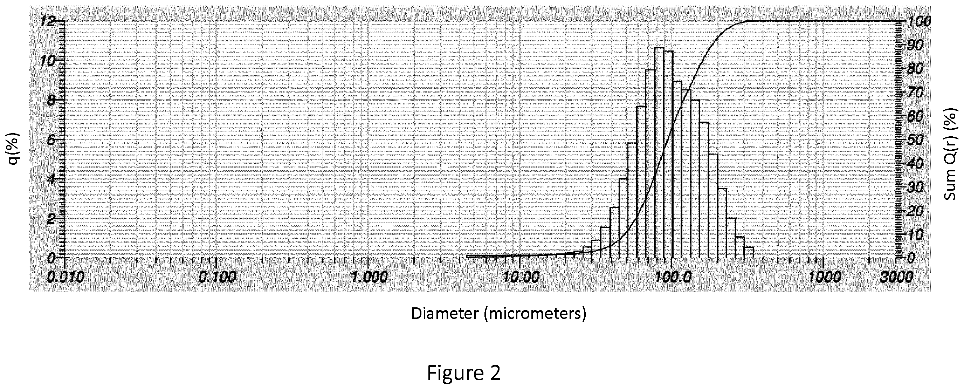

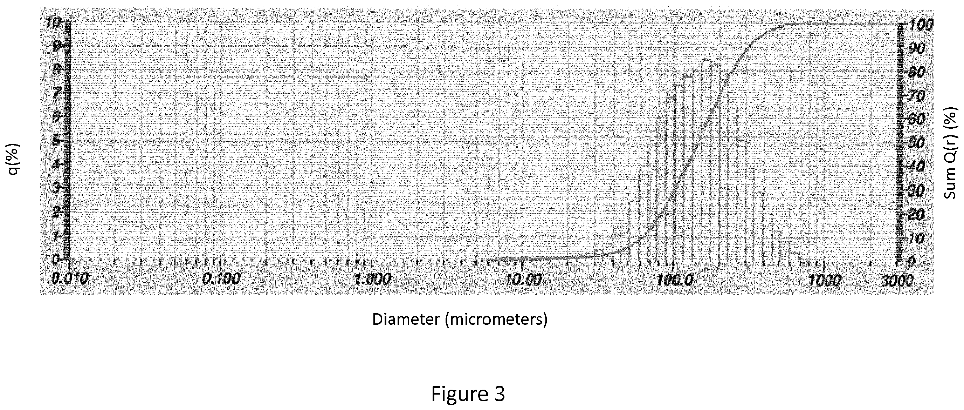

Furthermore, the volumetric distribution density of particles and the volumetric cumulative distribution versus diameter of both the starting and end products were measured as described above and, in each case, $d_{50}$ and $d_{10}$ were

determined. As mentioned above, Figure 2 illustrates the volumetric distribution density of particles, q (left hand y-axis) and the volumetric cumulative distribution Q(r) (right hand y-axis) versus diameter (x-axis) of particles of piroctone olamine which have not been recrystallized according to the invention, while Figure 3 illustrates volumetric distribution density of particles, q (left hand y-axis) and the volumetric cumulative distribution Q(r) (right hand y-axis) versus diameter (x-axis) of particles of piroctone olamine made according to Example 3, which have been subject to a recrystallization according to the invention.

The results were as follows:

|  | Starting Product (no recrystallization) | End Product (recrystallized) |
|---|---|---|
| $d_{50}$ | 90 | 145 |
| $d_{10}$ | 45 | 62 |
| Average w/L | 0.23 | 0.29 |

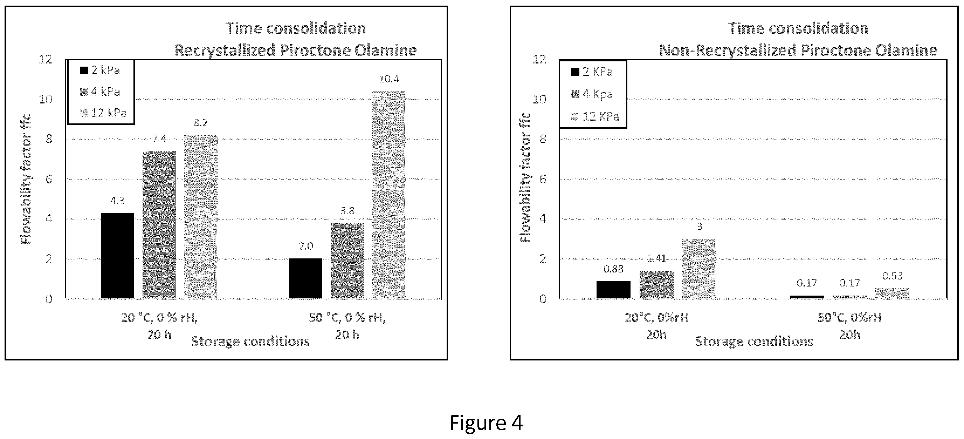

Figure 4 illustrates a comparison of the time consolidation of recrystallized piroctone olamine (left hand graph) and non-recrystallized piroctone olamine (right hand graph). Both sets of graphs comprise two sets of three measurements: a first set of measurements follows storage at 20 degrees Celsius, 0% relative humidity (rH) and a second set of measurements follows storage at 50 degrees Celsius, 0% relative humidity. Each set comprises three measurements, taken following consolidation after application of a 2kPa, a 4kPa and a 12kPa vertical pressure respectively. The flowability factor, $ff_c$, discussed above has been measured and is presented in the graphs. The results demonstrate that, under identical storage and consolidation conditions, the recrystallized piroctone olamine is significantly more flowable and therefore less liable to clumping than the non-recrystallized product.

Example 4

Raw materials:

| Raw material | Quantity | Equivalents |
|---|---|---|
| Piroctone Olamine | 100 kg | 1.0 |
| Isopropyl alcohol | 200 kg Two batches of 37 L | 2.74 |

Procedure:

1. Clean and dry the reactor.
2. Charge Isopropyl alcohol (200 kg) at 25-30 degrees Celsius.
3. Charge piroctone olamine (100 Kg) at 25-30 degrees Celsius.
4. Heat the mass to 70 degrees Celsius.
5. Maintain at 70-75 degrees Celsius for 30 minutes.
6. Check for dissolution (clear solution should be observed).
7. If incomplete dissolution, maintain at 70-75 degrees Celsius for another 30 minutes and check for clarity.
8. At 65-70°C add 500 g of piroctone olamine crystals for seeding.
9. Gradually cool the mass to 25-30 degrees Celsius over a period of 3-4 hrs.
10. Cool the mass to 8-10 degrees Celsius over a period of 2 hours.
11. Maintain at 8-10 degrees Celsius for 1 hour.
12. Centrifuge at 8-10 degrees Celsius, wash centrifuge with chilled (at 5-10 degrees Celsius) isopropyl alcohol (wash twice with 37 L).
13. Dry the wet filtercake in a cone dryer at a temperature below 45°C, then 1 hour at 60°C.
14. 92.0kg of wet product / 90.0kg dry product was obtained.

Average w/L, $d_{50}$ and $d_{10}$ were determined as described above. The results were as follows:

| | Starting Product (piroctone olamine - no recrystallization) | End Product (piroctone olamine - recrystallized) |
|---|---|---|
| $d_{50}$ | 90 | 163 |
| $d_{10}$ | 45 | 69 |
| Average w/L | 0.23 | 0.28 |

Example 5

Raw materials:

| Raw material | Quantity | Equivalents |
|---|---|---|
| Piroctone Olamine | 150 kg | 1.0 |
| Isopropyl alcohol | 200 kg Two batches of 37 L | 1.8 |

Procedure:

1. Clean and dry the reactor.
2. Charge Isopropyl alcohol (200 kg) at 25-30 degrees Celsius.
3. Charge piroctone olamine (150 Kg) at 25-30 degrees Celsius.
4. Heat the mass to 70 degrees Celsius.
5. Maintain at 70-75 degrees Celsius for 1 hr (a white dispersion is formed).
6. Gradually cool the mass to 25-30 degrees Celsius over a period of 8 hrs.
7. Cool the mass to 8-10 degrees Celsius over a period of 2 hours.
8. Maintain at 8-10 degrees Celsius for 1 hour.
9. Centrifuge at 8-10 degrees Celsius, wash centrifuge with chilled (at 5-10 degrees Celsius) isopropyl alcohol (wash twice with 37 L).
10. Filter the suspension at 8-10°C on a filter dryer, with chilled (at 5-10 degrees Celsius) isopropyl alcohol (wash twice with 37 L).
11. Dry the wet filter cake in a filter dryer at a temperature below 45°C, then 1 hour at 60°C.
12. 154 kg of wet product / 144 kg dry product was obtained.

Average w/L, $d_{50}$ and $d_{10}$ were determined as described above. The results were as follows:

| | Starting Product (piroctone olamine - no recrystallization) | End Product (piroctone olamine obtained by the above process) |
|---|---|---|
| $d_{50}$ | 90 | 280 |
| $d_{10}$ | 45 | 82 |
| Average w/L | 0.23 | 0.30 |

**Claims**

**1.** A process for the recrystallisation of piroctone olamine, comprising:

a) Dissolving piroctone olamine in a solvent comprising alcohol, wherein dissolving takes place at a dissolution temperature, which is a temperature above 50 degrees Celsius and below the boiling point of the alcohol, wherein the solvent comprises 90%wt or more of alcohol, and wherein the alcohol comprised in the solvent is selected from the group consisting of ethanol, isopropanol and mixtures thereof;
b) Cooling the solution from the dissolution temperature to a temperature between 1 and 15 degrees Celsius for a period up to 10 hours, preferably from 10 minutes to 10 hours;
c) Recovering crystals of piroctone olamine.

2. The process of claim 1, wherein in a) 1.5 to 4kg, preferably 2kg, of solvent are used per kilogramme of piroctone olamine.

3. The process of claim 1 or 2, wherein crystals of piroctone olamine are added for seeding.

4. The process of any preceding claim, wherein cooling takes place in two steps:

   i. In a first cooling step, cooling the solution from the dissolution temperature to a temperature between 20 and 40 degrees Celsius for a first cooling period which lasts up to 5 hours;
   ii. In a second cooling step, cooling the solution from a temperature between 20 and 40 degrees Celsius to a temperature between 1 and 15 degrees Celsius for a second cooling period which lasts up to 5 hours.

5. The process of any preceding claim, wherein the alcohol comprises isopropanol.

6. The process of any preceding claim, wherein the solvent consists of alcohol and preferably consists of isopropanol.

7. The process of any preceding claim, wherein the dissolution temperature is from 60 to 82.5 degrees Celsius, preferably from 70 to 75 degrees Celsius.

8. A process for the preparation of piroctone olamine crystals, comprising:

   a) Dispersing piroctone olamine in a solvent comprising alcohol, wherein dispersing takes place at a dispersion temperature, which is a temperature above 50 degrees Celsius and below the boiling point of the alcohol, wherein the solvent comprises 90%wt or more of alcohol, and wherein the alcohol comprised in the solvent is selected from the group consisting of ethanol, isopropanol and mixtures thereof;
   b) Cooling the dispersion from the dispersion temperature to a temperature between 1 and 15 degrees Celsius for a period up to 15 hours, preferably from 10 minutes to 15 hours, more preferably from 1 to 12 hours;
   c) Recovering crystals of piroctone olamine.

9. The process of claim 8, wherein in a) 1 to 3kg, preferably 1.0 to 1.5kg, more preferably 1.2 to 1.4kg, particularly preferably 1.33kg, of solvent are used per kilogramme of piroctone olamine.

10. The process of claim 8 or 9, wherein cooling takes place in two steps:

    i. In a first cooling step, cooling the dispersion from the dispersion temperature to a temperature between 20 and 40 degrees Celsius for a first cooling period which lasts up to 10 hours;
    ii. In a second cooling step, cooling the dispersion from a temperature between 20 and 40 degrees Celsius to a temperature between 1 and 15 degrees Celsius for a second cooling period which lasts up to 5 hours.

11. The process of any of claims 8 to 10, wherein the alcohol comprises isopropanol.

12. The process of any of claims 8 to 11, wherein the solvent consists of alcohol and preferably consists of isopropanol.

13. The process of any of claims 8 to 12, wherein the dispersion temperature is from 60 to 82.5 degrees Celsius, preferably from 70 to 75 degrees Celsius.

14. Piroctone olamine crystals having an average width to length ratio of greater than 1:4, obtainable by a process as defined in any of claims 1 to 13.

15. The piroctone olamine crystals of claim 14, having $d_{50}$ of greater than 110 micrometres preferably greater than 140 micrometres.

**16.** The piroctone olamine crystals of claim 14 or 15 having $d_{10}$ greater than 30 micrometres, preferably greater than 50 micrometres.

**Patentansprüche**

**1.** Verfahren zur Umkristallisation von Piroctone Olamine, umfassend:

a) Auflösen von Piroctone Olamine in einem Lösungsmittel, das Alkohol umfasst, wobei das Auflösen bei einer Auflösungstemperatur erfolgt, die über 50 Grad Celsius und unter dem Siedepunkt des Alkohols liegt, wobei das Lösungsmittel 90 Gew.-% oder mehr Alkohol umfasst und wobei der in dem Lösungsmittel enthaltene Alkohol aus der Gruppe bestehend aus Ethanol, Isopropanol und Mischungen davon ausgewählt ist;
b) Abkühlen der Lösung von der Auflösungstemperatur auf eine Temperatur zwischen 1 und 15 Grad Celsius für einen Zeitraum von bis zu 10 Stunden, vorzugsweise von 10 Minuten bis 10 Stunden;
c) Gewinnen von Kristallen von Piroctone Olamine.

**2.** Verfahren nach Anspruch 1, wobei in a) 1,5 bis 4 kg, vorzugsweise 2 kg, Lösungsmittel pro Kilogramm Piroctone Olamine verwendet werden.

**3.** Verfahren nach Anspruch 1 oder 2, wobei Kristalle von Piroctone Olamine zur Beimpfung zugegeben werden.

**4.** Verfahren nach einem der vorhergehenden Ansprüche, wobei das Abkühlen in zwei Schritten erfolgt:

i. in einem ersten Abkühlungsschritt Abkühlen der Lösung von der Auflösungstemperatur auf eine Temperatur zwischen 20 und 40 Grad Celsius für einen ersten Abkühlungszeitraum, der bis zu 5 Stunden dauert;
ii. in einem zweiten Abkühlungsschritt Abkühlen der Lösung von einer Temperatur zwischen 20 und 40 Grad Celsius auf eine Temperatur zwischen 1 und 15 Grad Celsius für einen zweiten Abkühlungszeitraum, der bis zu 5 Stunden dauert.

**5.** Verfahren nach einem der vorhergehenden Ansprüche, wobei der Alkohol Isopropanol umfasst.

**6.** Verfahren nach einem der vorhergehenden Ansprüche, wobei das Lösungsmittel aus Alkohol besteht und vorzugsweise aus Isopropanol besteht.

**7.** Verfahren nach einem der vorhergehenden Ansprüche, wobei die Auflösungstemperatur 60 bis 82,5 Grad Celsius, vorzugsweise 70 bis 75 Grad Celsius, beträgt.

**8.** Verfahren zur Herstellung von Piroctone-Olamine-Kristallen, umfassend:

a) Dispergieren von Piroctone Olamine in einem Lösungsmittel, das Alkohol umfasst, wobei das Dispergieren bei einer Dispergierungstemperatur erfolgt, die über 50 Grad Celsius und unter dem Siedepunkt des Alkohols liegt, wobei das Lösungsmittel 90 Gew.-% oder mehr Alkohol umfasst und wobei der in dem Lösungsmittel enthaltene Alkohol aus der Gruppe bestehend aus Ethanol, Isopropanol und Mischungen davon ausgewählt ist;
b) Abkühlen der Dispersion von der Dispergierungstemperatur auf eine Temperatur zwischen 1 und 15 Grad Celsius für einen Zeitraum bis zu 15 Stunden, vorzugsweise von 10 Minuten bis 15 Stunden, weiter bevorzugt von 1 bis 12 Stunden;
c) Gewinnen von Kristallen von Piroctone Olamine.

**9.** Verfahren nach Anspruch 8, wobei in a) 1 bis 3 kg, vorzugsweise 1,0 bis 1,5 kg, weiter bevorzugt 1,2 bis 1,4 kg, besonders bevorzugt 1,33 kg, Lösungsmittel pro Kilogramm Piroctone Olamine verwendet werden.

**10.** Verfahren nach Anspruch 8 oder 9, wobei das Abkühlen in zwei Schritten erfolgt:

i. in einem ersten Abkühlungsschritt Abkühlen der Dispersion von der Dispergierungstemperatur auf eine Temperatur zwischen 20 und 40 Grad Celsius für einen ersten Abkühlungszeitraum, der bis zu 10 Stunden dauert;
ii. in einem zweiten Abkühlungsschritt Abkühlen der Dispersion von einer Temperatur zwischen 20 und 40 Grad Celsius auf eine Temperatur zwischen 1 und 15 Grad Celsius für einen zweiten Abkühlungszeitraum, der bis zu 5 Stunden dauert.

**11.** Verfahren nach einem der Ansprüche 8 bis 10, wobei der Alkohol Isopropanol umfasst.

**12.** Verfahren nach einem der Ansprüche 8 bis 11, wobei das Lösungsmittel aus Alkohol besteht und vorzugsweise aus Isopropanol besteht.

**13.** Verfahren nach einem der Ansprüche 8 bis 12, wobei die Dispergierungstemperatur 60 bis 82,5 Grad Celsius, vorzugsweise 70 bis 75 Grad Celsius, beträgt.

**14.** Piroctone-Olamine-Kristalle mit einem durchschnittlichen Verhältnis von Breite zu Länge von mehr als 1:4, die durch ein Verfahren gemäß einem der Ansprüche 1 bis 13 erhältlich sind.

**15.** Piroctone-Olamine-Kristalle nach Anspruch 14 mit einem $d_{50}$ von mehr als 110 Mikrometer, vorzugsweise mehr als 140 Mikrometer.

**16.** Piroctone-Olamine-Kristalle nach Anspruch 14 oder 15 mit einem $d_{10}$ von mehr als 30 Mikrometer, vorzugsweise mehr als 50 Mikrometer.

**Revendications**

**1.** Procédé pour la recristallisation de piroctone olamine, comprenant :

a) la dissolution de piroctone olamine dans un solvant comprenant un alcool, la dissolution ayant lieu à une température de dissolution, qui est une température supérieure à 50 degrés Celsius et inférieure au point d'ébullition de l'alcool, le solvant comprenant 90 % en poids ou plus d'alcool, et l'alcool compris dans le solvant étant choisi dans le groupe constitué par l'éthanol, l'isopropanol et des mélanges correspondants ;
b) le refroidissement de la solution depuis la température de dissolution jusqu'à une température entre 1 et 15 degrés Celsius pendant une période jusqu'à 10 heures, préférablement de 10 minutes à 10 heures ;
c) la récupération de cristaux de piroctone olamine.

**2.** Procédé selon la revendication 1, en a) 1,5 à 4 kg, préférablement 2 kg, de solvant étant utilisés par kilogramme de piroctone olamine.

**3.** Procédé selon la revendication 1 ou 2, des cristaux de piroctone olamine étant ajoutés pour un ensemencement.

**4.** Procédé selon une quelconque revendication précédente, le refroidissement ayant lieu en deux étapes :

i. dans une première étape de refroidissement, refroidissement de la solution depuis la température de dissolution jusqu'à une température entre 20 et 40 degrés Celsius pendant une première période de refroidissement qui dure jusqu'à 5 heures ;
ii. dans une deuxième étape de refroidissement, refroidissement de la solution depuis une température entre 20 et 40 degrés Celsius jusqu'à une température entre 1 et 15 degrés Celsius pendant une deuxième période de refroidissement qui dure jusqu'à 5 heures.

**5.** Procédé selon une quelconque revendication précédente, l'alcool comprenant l'isopropanol.

**6.** Procédé selon une quelconque revendication précédente, le solvant étant constitué d'alcool et préférablement constitué d'isopropanol.

**7.** Procédé selon une quelconque revendication précédente, la température de dissolution étant de 60 à 82,5 degrés Celsius, préférablement de 70 à 75 degrés Celsius.

**8.** Procédé pour la préparation de cristaux de piroctone olamine, comprenant :

a) la dispersion de piroctone olamine dans un solvant comprenant un alcool, la dispersion ayant lieu à une température de dispersion, qui est une température supérieure à 50 degrés Celsius et inférieure au point d'ébullition de l'alcool, le solvant comprenant 90 % en poids ou plus d'alcool, et l'alcool compris dans le solvant étant choisi dans le groupe constitué par l'éthanol, l'isopropanol et des mélanges correspondants ;

b) le refroidissement de la dispersion depuis la température de dispersion jusqu'à une température entre 1 et 15 degrés Celsius pendant une période jusqu'à 15 heures, préférablement de 10 minutes à 15 heures, plus préférablement de 1 à 12 heures ;

c) la récupération de cristaux de piroctone olamine.

9. Procédé selon la revendication 8, en a) 1 à 3 kg, préférablement 1,0 à 1,5 kg, plus préférablement 1,2 à 1,4 kg, particulièrement préférablement 1,33 kg, de solvant étant utilisés par kilogramme de piroctone olamine.

10. Procédé selon la revendication 8 ou 9, le refroidissement ayant lieu en deux étapes :

i. dans une première étape de refroidissement, refroidissement de la dispersion depuis la température de dispersion jusqu'à une température entre 20 et 40 degrés Celsius pendant une première période de refroidissement qui dure jusqu'à 10 heures ;

ii. dans une deuxième étape de refroidissement, refroidissement de la dispersion depuis une température entre 20 et 40 degrés Celsius jusqu'à une température entre 1 et 15 degrés Celsius pendant une deuxième période de refroidissement qui dure jusqu'à 5 heures.

11. Procédé selon l'une quelconque des revendications 8 à 10, l'alcool comprenant l'isopropanol.

12. Procédé selon l'une quelconque des revendications 8 à 11, le solvant étant constitué d'alcool et préférablement constitué d'isopropanol.

13. Procédé selon l'une quelconque des revendications 8 à 12, la température de dispersion étant de 60 à 82,5 degrés Celsius, préférablement de 70 à 75 degrés Celsius.

14. Cristaux de piroctone olamine ayant un rapport de largeur moyenne sur longueur supérieur à 1 : 4, pouvant être obtenus par un procédé tel que défini dans l'une quelconque des revendications 1 à 13.

15. Cristaux de piroctone olamine selon la revendication 14, ayant un $d_{50}$ supérieur à 110 micromètres, préférablement supérieur à 140 micromètres.

16. Cristaux de piroctone olamine selon la revendication 14 ou 15 ayant un $d_{10}$ supérieur à 30 micromètres, préférablement supérieur à 50 micromètres.

Figure 1

Figure 2

Figure 3

Figure 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 2234009 A1
- DE 1795270 A1
- EP 0710650 A2
- DE 3626210 C1
- CN 1907971 A

**Non-patent literature cited in the description**

- **SCHULZE, D.** Pulver und Schüttgüter. Springer, 2009